# EUROPEAN PATENT APPLICATION

(11) **EP 3 744 678 A1**
(43) Date of publication of application: **02.12.2020**
(21) Application number: 19760112.3
(22) Date of filing: 28.02.2019
(51) Int. Cl.: B81B 3/00, B01J 19/00, G01N 37/00, C12M 1/34

(54) **MICROFLUIDIC CHANNEL DEVICE**

(30) Priority: 02.03.2018 JP 2018037511
(71) Applicant: FUJIFILM Corporation, Tokyo 106-8620 (JP)
(72) Inventor: MIYOSHI, Hayato, Ashigarakami-gun, Kanagawa 258-8577 (JP); ITO, Koju, Ashigarakami-gun, Kanagawa 258-8577 (JP); OBA, Takahiro, Ashigarakami-gun, Kanagawa 258-8577 (JP); WAKABAYASHI, Akira, Ashigarakami-gun, Kanagawa 258-8577 (JP)
(74) Representative: Hoffmann Eitle
(86) International application number: PCT/JP2019/007907
(87) International publication number: WO 2019/168118

(57) **Abstract**

Provided is a microchannel device including a first microchannel that is formed in a first channel member, a second microchannel that is formed in a second channel member and at least a portion of which overlaps the first microchannel in plan view with a step portion formed between the first microchannel and the second microchannel, a porous membrane that has a plurality of holes penetrating the porous membrane in a thickness direction and is disposed between the first channel member and the second channel member to partition the first microchannel and the second microchannel, and a reinforcing member that is provided between the first channel member or the second channel member and the porous membrane, is higher in stiffness than the porous membrane, and reinforces at least a portion of the porous membrane that faces the step portion.

## Description

### Technical Field

The present disclosure relates to a microchannel device.

### Background Art

A device having a channel, of which the width is of the order of micrometers and which is called a microchannel, (hereinafter, referred to as "microchannel device") has been known.

For example, JP5415538B discloses, as a microchannel device, an organ mimic device having a first central micro channel and a second central micro channel partitioned by a porous membrane.

### SUMMARY OF THE INVENTION

### Technical Problem

In the organ mimic device disclosed in JP5415538B, the first central micro channel on an upper side and a second central micro channel on a lower side overlap each other in plan view at central portions thereof, an inlet port and an outlet port are separated from each other, and a step portion is formed at a junction portion at which the first central micro channel on the upper side and the second central micro channel on the lower side join each other in plan view.

Since the step portion is formed between the first central micro channel and the second central micro channel, for example, in a case where a cell suspension is caused to flow into the first micro channel such that a cell layer is formed on a surface of the porous membrane, the porous membrane is bent due to the liquid pressure of the cell suspension and thus a gap is formed between the step portion and the porous membrane. In addition, even after the formation of the cell layer, in a case where a test solution (for example, blood diluent or liquid containing tracer such as FITC-MicroBeads) is caused to flow into the first central micro channel to perform a test, a gap is formed between the step portion and the porous membrane due to the liquid pressure of the test solution.

At this time, cells in the cell suspension or red blood cells or the tracer in the test solution may flow into the gap between the step portion and the porous membrane and be caught in the gap and a portion of the cell layer, the red blood cells, or the tracer may be positioned in the second central micro channel on the lower side. In this case, since cells, red blood cells, or a tracer seems to have passed through the porous membrane from the first central micro channel and to have leaked into the second central micro channel in a case where, for example, a permeability test for the cells, the red blood cells, or the tracer is performed using the organ mimic device, it is difficult to perform the permeability test accurately.

The present disclosure provides a microchannel device with which it is possible to suppress formation of a gap between a step portion and a porous membrane and it is possible to restrain cells, red blood cells, or a tracer from flowing into the gap, the step portion being formed between a first microchannel and a second microchannel.

### Solution to Problem

According to a first aspect of the present disclosure, there is provided a microchannel device comprising a first microchannel that is formed in a first channel member, a second microchannel that is formed in a second channel member and at least a portion of which overlaps the first microchannel in plan view, the second microchannel having a step portion formed between the first microchannel and the second microchannel, a porous membrane that has a plurality of holes penetrating the porous membrane in a thickness direction and is disposed between the first channel member and the second channel member to partition the first microchannel and the second microchannel, and a reinforcing member that is provided between the first channel member or the second channel member and the porous membrane, is higher in stiffness than the porous membrane, and reinforces at least a portion of the porous membrane that faces the step portion.

According to the above-described configuration, the step portion is formed between the first microchannel and the second microchannel and the portion of the porous membrane that faces the step portion is reinforced by the reinforcing member. Therefore, in a case where a cell suspension is caused to flow into the first microchannel or the second microchannel and a cell layer is formed on a surface of the porous membrane, formation of a gap that is formed between the step portion and the porous membrane due to the porous membrane bent by the liquid pressure of the cell suspension can be suppressed and thus it is possible to restrain cells from flowing into the gap.

Similarly, in a case where a test solution is caused to flow into the first microchannel or the second microchannel, formation of a gap that is formed between the step portion and the porous membrane due to the porous membrane bent by the liquid pressure of the test solution can be suppressed and thus it is possible to restrain red blood cells or a tracer from flowing into the gap.

According to a second aspect of the present disclosure, in the microchannel device related to the first aspect, the first microchannel and the second microchannel may be partially separated from each other in plan view and the step portion may be formed at a junction portion at which the first microchannel and the second microchannel join each other in plan view.

According to the above-described configuration, at the step portion formed at the junction portion at which the first microchannel and the second microchannel join each other in plan view, formation of a gap between the step portion and the porous membrane can be suppressed since the porous membrane is reinforced by the reinforcing member.

According to a third aspect of the present disclosure, in the microchannel device related to the first aspect, a width of the first microchannel may be smaller than a width of the second microchannel and the step portion may be formed by a difference between the width of the first microchannel and the width of the second microchannel.

According to the above-described configuration, at the step portion formed by the difference between the width of the first microchannel and the width of the second microchannel, formation of a gap between the step portion and the porous membrane can be suppressed since the porous membrane is reinforced by the reinforcing member.

According to a fourth aspect of the present disclosure, in the microchannel device related to any one of the first to third aspects, the reinforcing member may have a size that covers the entire porous membrane and a slit may be formed in the reinforcing member at a portion where the porous membrane faces the first microchannel or the second microchannel.

According to the above-described configuration, the porous membrane can be further reinforced with the reinforcing member covering the entire porous membrane. In addition, since the slit is formed in the reinforcing member, in a case where cells, red blood cells, or a tracer moves between the first microchannel and the second microchannel through the porous membrane, it is possible to restrain the reinforcing member from inhibiting the movement of the cells, the red blood cells, or the tracer.

According to a fifth aspect of the present disclosure, in the microchannel device related to the fourth aspect, a width of the slit of the reinforcing member may be equal to or smaller than a width of each of the first microchannel and the second microchannel.

According to the above-described configuration, the width of the slit of the reinforcing member is equal to or smaller than the width of each of the first microchannel and the second microchannel. Therefore, in comparison with a case where the width of the slit of the reinforcing member is larger than the width of each of the first microchannel and the second microchannel, it is possible to further suppress formation of a gap between the step portion and the porous membrane, the step portion being formed between the first microchannel and the second microchannel.

According to a sixth aspect of the present disclosure, in the microchannel device related to any one of the first to fifth aspects, the reinforcing member may be a membrane member formed of polyethylene terephthalate.

According to the above-described configuration, since the reinforcing member is the membrane member formed of polyethylene terephthalate which is not likely to affect cell culture, it is possible to restrain components contained in the reinforcing member from affecting cells or the like in the first microchannel or the second microchannel.

According to a seventh aspect of the present disclosure, in the microchannel device related to any one of the first to fifth aspects, the reinforcing member may be a membrane member formed of polypropylene.

According to the above-described configuration, since the reinforcing member is the membrane member formed of polypropylene which is not likely to affect cell culture, it is possible to restrain components contained in the reinforcing member from affecting cells or the like in the first microchannel or the second microchannel.

According to an eighth aspect of the present disclosure, in the microchannel device related to any one of the first to seventh aspects, a thickness of the reinforcing member may be equal to or greater than 12 µm.

According to the above-described configuration, since the thickness of the reinforcing member formed of polyethylene terephthalate is equal to or greater than 12 µm, the stiffness of the reinforcing member can be increased in comparison with a configuration in which the thickness of the reinforcing member is smaller than 12 µm and thus it is possible to reinforce the porous membrane more strongly by means of the reinforcing member.

According to a ninth aspect of the present disclosure, in the microchannel device related to any one of the first to eighth aspects, a thickness of the reinforcing member may be smaller than a depth of each of the first microchannel and the second microchannel.

According to the above-described configuration, the thickness of the reinforcing member is smaller than the depth of each of the first microchannel and the second microchannel. Therefore, in comparison with a case where the thickness of the reinforcing member is larger than the depth of each of the first microchannel and the second microchannel, it is possible to restrain the reinforcing member from inhibiting the seeding of cells onto the porous membrane in a case where a cell suspension is caused to flow into the first microchannel or the second microchannel and a cell layer is formed on a surface of the porous membrane.

### Effects of Invention

According to the present disclosure, it is possible to suppress formation of a gap between the step portion and the porous membrane and thus it is possible to restrain cells, red blood cells, or a tracer from flowing into the gap, the step portion formed between the first microchannel and the second microchannel.

### BRIEF DESCRIPTION OF THE DRAWINGS

Fig. 1 is a perspective view showing the structure of the entire microchannel device in a first embodiment.
Fig. 2 is an exploded perspective view showing the structure of the entire microchannel device in the first embodiment.
Fig. 3 is a see-through view showing the microchannel device in the first embodiment as seen in plan view.
Fig. 4 is a sectional view taken along line A-A in Fig. 3.
Fig. 5 is a sectional view taken along line B-B in Fig. 3.
Fig. 6 is a plan view showing a porous membrane of the microchannel device in the first embodiment.
Fig. 7 is a sectional view taken along line C-C in Fig. 6.
Fig. 8 is an exploded perspective view showing the structure of the entire microchannel device in a second embodiment.
Fig. 9 is a see-through view showing the microchannel device in the second embodiment as seen in plan view.
Fig. 10A is an enlarged plan view showing a state where a cell suspension is caused to flow into a microchannel device in an example.
Fig. 10B is a sectional view taken along line D-D in Fig. 10A.
Fig. 11A is an enlarged plan view showing a state where a cell suspension is caused to flow into a microchannel device in a comparative example.
Fig. 11B is a sectional view taken along line E-E in Fig. 11A.

### DESCRIPTION OF THE PREFERRED EMBODIMENTS

### (First Embodiment)

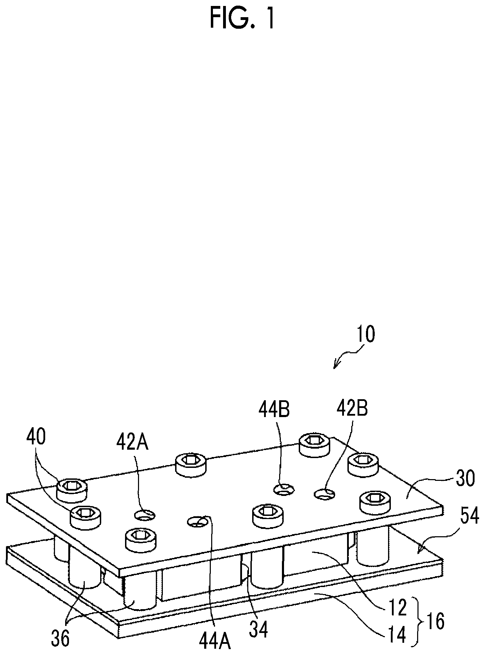

Hereinafter, a first embodiment of the present disclosure will be described with reference to Figs. 1 to 7. Note that the following embodiments are examples of the present disclosure and are not intended to limit the scope of the present disclosure. In addition, to facilitate the description of each configuration, the dimensions of each configuration in the drawings have been appropriately changed. Therefore, the scale of the drawings is different from that of the real scale.

### <Channel Unit>

As shown in Fig. 1, a microchannel device 10 of the present embodiment has a channel unit 16 composed of a first channel member 12 and a second channel member 14 laminated in a thickness direction. For example, it is preferable that the first channel member 12 is formed of an elastic transparent material such as polydimethylsiloxane (PDMS) and the second channel member 14 is formed of a rigid transparent material such as a cycloolefin polymer (COP).

Note that, examples of materials constituting the first channel member 12 and the second channel member 14 include epoxy resin, urethane resin, and styrenic thermoplastic elastomers, olefinic thermoplastic elastomers, acrylic thermoplastic elastomers, and polyvinyl alcohol in addition to polydimethylsiloxane (PDMS) and cycloolefin polymers (COP).

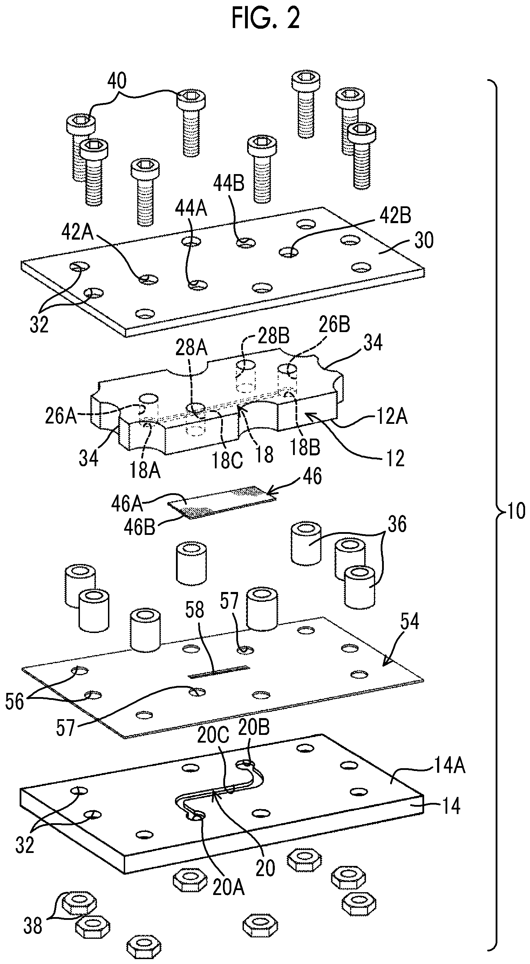

As shown in Fig. 2, a first microchannel 18 is formed in a lower surface of the first channel member 12, that is, in a facing surface 12A facing the second channel member 14. The first microchannel 18 has an inflow port 18A, an outflow port 18B, and a channel portion 18C through which the inflow port 18A and the outflow port 18B communicate with each other and that extends approximately linearly.

Similarly, a second microchannel 20 is formed in an upper surface of the second channel member 14, that is, a facing surface 14A facing the first channel member 12. The second microchannel 20 has an inflow port 20A, an outflow port 20B, and a channel portion 20C through which the inflow port 20A and the outflow port 20B communicate with each other and that extends approximately linearly.

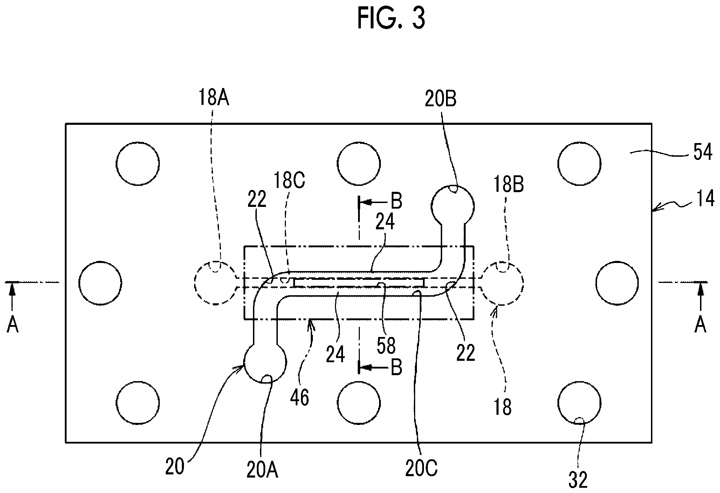

Here, as shown in Fig. 3, the inflow port 18A and the outflow port 18B of the first microchannel 18 are provided at positions separated from the inflow port 20A and the outflow port 20B of the second microchannel 20 in plan view. Meanwhile, the channel portion 18C of the first microchannel 18 is provided at a position overlapping the channel portion 20C of the second microchannel 20 in plan view.

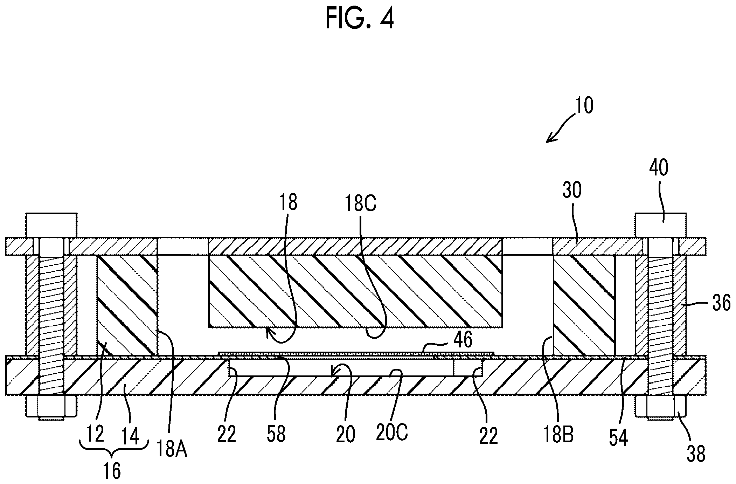

Accordingly, as shown in Figs. 3 and 4, step portions 22 are formed at a junction portion between the first microchannel 18 and the second microchannel 20, that is, at a position between the inflow ports 18A and 20A and the channel portions 18C and 20C and at a position between the outflow ports 18B and 20B and the channel portions 18C and 20C, respectively.

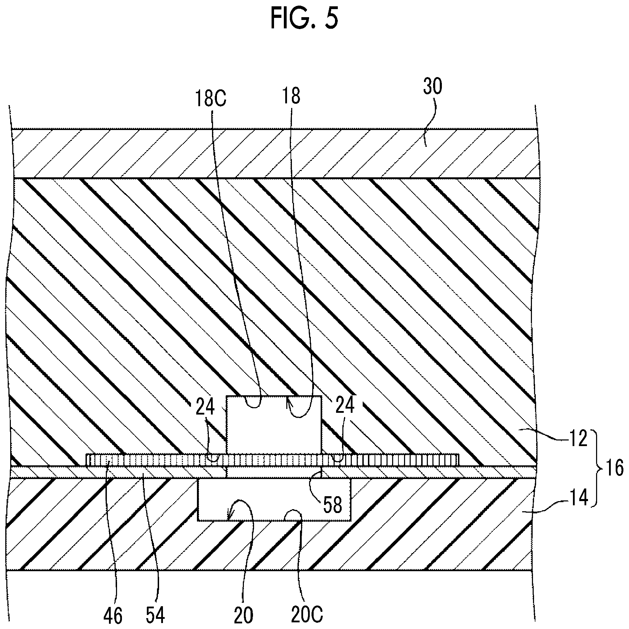

In addition, as shown in Figs. 3 and 5, the width of the channel portion 18C of the first microchannel 18 is smaller than the width of the channel portion 20C of the second microchannel 20 and step portions 24 are formed by the difference between the width of the channel portion 18C and the width of the channel portion 20C.

As shown in Fig. 2, through-holes 26A and 26B that penetrate the first channel member 12 in the thickness direction and of which the lower ends communicate with the inflow port 18A and the outflow port 18B of the first microchannel 18 and through-holes 28A and 28B that penetrate the first channel member 12 in the thickness direction and of which the lower ends communicate with the inflow port 20A and the outflow port 20B of the second microchannel 20 are formed in the first channel member 12.

In addition, a holding plate 30 having a size that covers the entire upper surface of the first channel member 12 is provided above the first channel member 12. A plurality of (eight in present embodiment) bolt holes 32 are formed at corresponding positions in each of the holding plate 30 and the second channel member 14, the bolt holes 32 penetrating the holding plate 30 and the second channel member 14 in the thickness direction.

Meanwhile, on an outer peripheral surface of the first channel member 12, recess portions 34 are formed at positions corresponding to the bolt holes 32 and a plurality of (eight in present embodiment) spacers 36 defining a gap between the holding plate 30 and the second channel member 14 are provided outward of the recess portions 34 of the channel unit 16.

The spacers 36 are cylindrical members each of which has an inner diameter approximately equal to the inner diameter of each bolt hole 32 and are disposed at positions corresponding to the bolt holes 32. In addition, the holding plate 30 and the second channel member 14 are bonded with a reinforcing member 54 (which will be described later) by a plurality of bolts 40, the bolts 40 being inserted into the bolt holes 32 and the spacers 36 and fixed by nuts 38.

Note that, through-holes 42A, 42B, 44A, and 44B that communicate with the through-holes 26A, 26B, 28A, and 28B of the first channel member 12 respectively are formed in the holding plate 30. Tubes (not shown) are respectively connected to the through-holes 42A, 42B, 44A, and 44B, a solution, a cell suspension, or the like flows into the first microchannel 18 and the second microchannel 20 through the tubes and the solution, the cell suspension, or the like flows out from the first microchannel 18 and the second microchannel 20.

### <Porous Membrane>

A porous membrane 46 is disposed between the facing surfaces 12A and 14A of the first channel member 12 and the second channel member 14. The porous membrane 46 is formed of, for example, a hydrophobic polymer that can be dissolved in a hydrophobic organic solvent. Note that, the hydrophobic organic solvent is liquid of which the solubility in water at 25°C is 10 (g/100g water) or less.

Examples of the hydrophobic polymer include polymers such as polystyrene, polyacrylate, polymethacrylate, polyacrylamide, polymethacrylamide, polyvinyl chloride, polyvinylidene chloride, polyvinylidene fluoride, polyhexafluoropropene, polyvinyl ether, polyvinylcarbazole, polyvinyl acetate, polytetrafluoroethylene, polyester (for example, polyethylene terephthalate, polyethylene naphthalate, polyethylene succinate, polybutylene succinate, polylactic acid, poly-3-hydroxybutyrate or like), polylactone (for example, polycaprolactone or like), polyamide or polyimide (for example, nylon, polyamic acid, or like), polyurethane, polyurea, polybutadiene, polycarbonate, polyaromatics, polysulfone, polyethersulfone, polysiloxane derivatives, and cellulose acylate (for example, triacetyl cellulose, cellulose acetate propionate, cellulose acetate butyrate).

These polymers may be homopolymers, copolymers, polymer blends or polymer alloys as necessary in the viewpoint of solubility in solvents, optical properties, electrical properties, membrane strength, elasticity and the like. In addition, these polymers may be used alone or in combination of two or more. Note that, the material of the porous membrane 46 is not limited to the hydrophobic polymer and various materials can be selected in the viewpoint of cell adhesiveness or the like.

An upper surface 46A and a lower surface 46B (hereinafter, upper 46Aand lower surface 46B may be collectively referred to as "main surfaces") of the porous membrane 46 have sizes that approximately cover the channel portions 18C and 20C of the first microchannel 18 and the second microchannel 20 and partition the first microchannel 18 and the second microchannel 20. Specifically, the upper surface 46A of the porous membrane 46 faces the first microchannel 18 and the lower surface 46B of the porous membrane 46 faces the second microchannel 20.

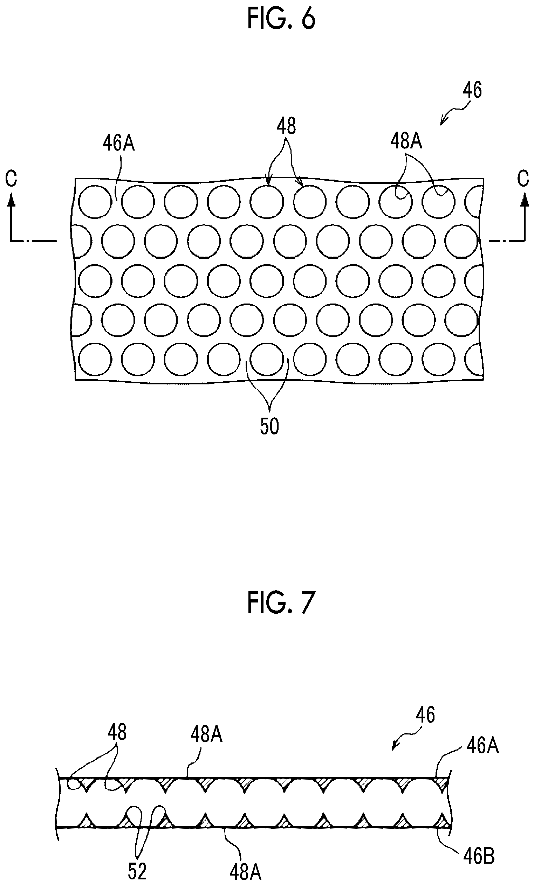

As shown in Figs. 6 and 7, a plurality of holes 48 penetrating the porous membrane 46 in the thickness direction are formed in the porous membrane 46 and openings 48A of the holes 48 are provided in the upper surface 46A and the lower surface 46B of the porous membrane 46. In addition, as shown in Fig. 6, each opening 48A has a circular shape in plan view. The openings 48A are provided while being separated from each other and a flat portion 50 extends between adjacent openings 48A. Note that, the shape of the opening 48A is not limited to a circular shape and may be a polygonal shape or an oval shape.

The plurality of openings 48A are arranged regularly and are arranged in a honeycomb shape in the present embodiment, for example. Being arranged in a honeycomb shape is being arranged in units of shapes like hexagonal parallelogons (preferably regular hexagons) or similar shapes such that the centers of the openings 48A are positioned at vertexes of the shapes and intersection points between diagonal lines. Here, the meaning of "the centers of the openings" is the centers of the openings 48A in plan view.

Note that, the openings 48A may not be arranged in a honeycomb shape and may be arranged in a lattice shape or a face-centered lattice shape. Being arranged in a lattice shape is being arranged in units of shapes like parallelograms (it is matter of course that parallelograms include squares, rectangles, and rhombuses) (preferably, squares) such that the centers of the openings are positioned at vertexes of the shapes. Being arranged in a face-centered lattice shape is being arranged in units of shapes like parallelograms (it is matter of course that parallelograms include squares, rectangles, and rhombuses) (preferably, squares) such that the centers of the openings are positioned at vertexes of the shapes and intersection points between diagonal lines.

As shown in Fig. 7, each of the holes 48 of the porous membrane 46 has a shape obtained by cutting an upper end and a lower end of a sphere. In addition, adjacent holes 48 communicate with each other through a communication hole 52 inside the porous membrane 46.

It is preferable that one hole 48 communicates with all of the holes 48 adjacent thereto and in a case where the openings 48A of the plurality of holes 48 are arranged in a honeycomb shape as in the present embodiment, one hole 48 communicates with six holes 48 adjacent thereto through six communication holes 52. Note that, each of the holes 48 may have a barrel shape, a circular columnar shape, a polygonal columnar shape, or the like and each communication hole 52 may be a tubular void connecting adjacent holes 48 to each other.

Note that, it is preferable that at least a region on the main surfaces of the porous membrane 46 on which cells are seeded is coated by at least one of fibronectin, collagen (for example, type I collagen, type IV collagen, or type V collagen), laminin, vitronectin, gelatin, perlecan, nidogen, proteoglycan, osteopontin, tenascin, nephronectin, a basement membrane matrix, or polylysine. In addition, it is also preferable that the insides of the holes 48 of the porous membrane 46 are coated by at least one of those described above. Since the porous membrane 46 is coated, it is possible to enhance cell adhesiveness.

In addition, since the porous membrane 46 is a foothold to which cells are bonded and at which the cells are propagated, the higher the opening ratio of the porous membrane 46 is and the thinner the porous membrane 46 is, the more active at least one of cell-cell interaction between cells on one surface and cells on the other surface, that is, information exchange made by humoral factors or cell-cell contact is. The more active the cell-cell interaction at the time of cell propagation at the main surfaces of the porous membrane 46 is, the more similar the function of a manufacturable model is to a tissue in a living body.

Examples of a method of producing the porous membrane 46 in which the holes 48 are formed include an etching method, a sandblast method, a press molding method and the like in addition to a nanoprinting method, a dew condensation method. The nanoprinting method is a method of producing the holes 48 by pouring a material constituting the porous membrane 46 into a mold having an uneven shape or pressing the mold against the material constituting the porous membrane 46. In addition, the dew condensation method is a method of forming the holes 48 by causing dew condensation on a surface of the material constituting the porous membrane 46 and using water droplets as a mold.

In the case of the dew condensation method, it is possible to make the porous membrane 46 thin and the void volume or the opening ratio of the openings 48A large in comparison with other methods and it is possible to provide the communication holes 52 in the porous membrane 46. Therefore, in the present embodiment, the porous membrane 46 is manufactured by using the dew condensation method. Details of the dew condensation method are described in, for example, JP4945281B, JP5422230B, JP2011-074140A, and JP5405374B.

### <Reinforcing Member>

As shown in Figs. 1 to 5, a reinforcing member 54 having a higher stiffness than the porous membrane 46 is disposed between the porous membrane 46 and the facing surface 14A of the second channel member 14. The reinforcing member 54 is, for example, a membrane member formed of polyethylene terephthalate and has a size that covers the entire porous membrane 46. Specifically, the reinforcing member 54 has approximately the same size as the facing surface 14A of the second channel member 14 and covers a portion of the porous membrane 46 that faces the step portions 22 and 24 formed between the first microchannel 18 and the second microchannel 20.

In addition, it is preferable that the thickness of the reinforcing member 54 is equal to or greater than 12 µm and is smaller than the depth of the first microchannel 18 or the second microchannel 20. Specifically, it is preferable that the thickness of the reinforcing member 54 is equal to or greater than 12 µm and equal to or smaller than 400 µm and it is more preferable that the thickness of the reinforcing member 54 is equal to or greater than 12 µm and is equal to or smaller than 200 µm.

In addition, the stiffness of the reinforcing member 54 can be evaluated by measuring the amount of deformation of the reinforcing member 54 that is made in a case where a steel ball is placed on the reinforcing member 54. Specifically, a plate that is formed of stainless steel (SUS), has a thickness of 3 mm, and in which a hole of Φ50 mm is formed is prepared and four sides of the reinforcing member 54 having a size of 70 mm square are fixed onto the plate by means of double-sided tape (No. 5000NS manufactured by NITTO DENKO CORPORATION). Then, a steel ball of which the diameter is 11 mm and the weight is 5.5 g is placed on the center portion of the hole with the reinforcing member 54 interposed therebetween and the amount of downward deformation of the reinforcing member 54 at the center portion of the hole at that time is measured from a position below the plate by means of a laser displacement gauge (LK-G85 manufactured by Keyence Corporation).

In the case of the above-described evaluation method, the higher the stiffness of the reinforcing member 54 is, the larger the amount of deformation is and it is preferable that the amount of deformation of the reinforcing member 54 is equal to or smaller than 3 mm and it is more preferable that the amount of deformation is equal to or smaller than 1 mm in a case where the above-described evaluation method is used.

Note that, it is sufficient that the reinforcing member 54 has a stiffness higher than the stiffness of at least the porous membrane 46 and is formed of a material that is not likely to affect cell culture. Examples of a material that is not likely to affect cell culture include silicone materials such as polydimethylsiloxane (PDMS), polystyrene, polyethylene naphthalate (PEN), polypropylene, cycloolefin polymer (COP), polyethylene (PE), and the like in addition to polyethylene terephthalate. In addition, the required thickness of the reinforcing member 54 is appropriately determined depending on the material of the reinforcing member 54.

As shown in Fig. 2, a plurality of (eight in the present embodiment) bolt holes 56 are formed in the reinforcing member 54 at positions corresponding to the bolt holes 32 of the second channel member 14 and the reinforcing member 54 is bonded to the holding plate 30 and the second channel member 14 by means of the bolts 40. In addition, through-holes 57 are formed in the reinforcing member 54 at positions corresponding to the inflow port 20A and the outflow port 20B of the second microchannel 20.

In addition, a slit 58 is formed in the reinforcing member 54 at a portion where the porous membrane 46 faces the first microchannel 18 and the second microchannel 20. Specifically, as shown in Fig. 3, the slit 58 is provided at a position where the slit 58 overlaps the channel portion 18C of the first microchannel 18 and the channel portion 20C of the second microchannel 20 in plan view and the width of the slit 58 is approximately the same as the width of the channel portion 18C of the first microchannel 18. Note that, it is sufficient that the width of the slit 58 is equal to or smaller than the width of the channel portion 18C of the first microchannel 18 at least.

### <Action and Effect>

According to the present embodiment, the step portions 22 are formed at the junction portion between the first microchannel 18 and the second microchannel 20 and the step portions 24 are formed by the difference between the width of the channel portion 18C of the first microchannel 18 and the width of the channel portion 20C of the second microchannel 20. In addition, a portion of the porous membrane 46 that faces the step portions 22 and 24 is reinforced by being covered by the reinforcing member 54.

Therefore, even in a case where the liquid pressure of a cell suspension is applied to the porous membrane 46 in a case where the cell suspension is caused to flow into the first microchannel 18 and a cell layer is formed on the upper surface 46A of the porous membrane 46, the porous membrane 46 can be restrained from being bent toward the second microchannel 20 side since the reinforcing member 54 is provided. Accordingly, it is possible to suppress formation of a gap between the step portions 22 and 24 and the porous membrane 46 and thus it is possible to restrain cells from flowing into the gap.

In addition, in the present embodiment, since the reinforcing member 54 has a size that covers the entire porous membrane 46, the porous membrane 46 can be reinforced more in comparison with a configuration in which the reinforcing member 54 has a size that covers only a portion of the porous membrane 46.

Furthermore, the slit 58 is formed in the reinforcing member 54 at the portion where the porous membrane 46 faces the first microchannel 18 and the second microchannel 20. Therefore, in the case of a permeability test for cells, red blood cells, or a tracer, the cells, the red blood cells, or the tracer can move between the first microchannel 18 and the second microchannel 20 through the slit 58 of the reinforcing member 54 and the porous membrane 46 and thus it is possible to restrain the reinforcing member 54 from inhibiting the movement of the cells, the red blood cells, or the tracer.

Particularly, in the present embodiment, the width of the slit 58 of the reinforcing member 54 is set to be approximately the same as the width of the channel portion 18C of the first microchannel 18. Therefore, in comparison with a case where the width of the slit 58 of the reinforcing member 54 is larger than the width of the channel portion 18C, it is possible to suppress formation of a gap between the step portions 24 and the porous membrane 46. In addition, in comparison with a case where the width of the slit 58 of the reinforcing member 54 is smaller than the width of the channel portion 18C, it is possible to restrain the reinforcing member 54 from inhibiting the movement of the cells, the red blood cells, or the tracer.

In addition, according to the present embodiment, the reinforcing member 54 is a membrane member formed of polyethylene terephthalate or the like which is biocompatible. Therefore, it is possible to restrain components contained in the reinforcing member 54 from affecting cells or the like in the first microchannel 18 or the second microchannel 20.

In addition, the thickness of the reinforcing member 54 is equal to or greater than 12 µm and is smaller than the depth of the first microchannel 18 or the second microchannel 20. Therefore, the stiffness of the reinforcing member 54 can be increased in comparison with a configuration in which the thickness of the reinforcing member 54 is smaller than 12 µm and thus it is possible to reinforce the porous membrane 46 more strongly by means of the reinforcing member 54.

Furthermore, in comparison with a case where the thickness of the reinforcing member 54 is larger than the depth of each of the first microchannel 18 and the second microchannel 20, it is possible to restrain the reinforcing member 54 from inhibiting the seeding of cells onto the porous membrane 46 in a case where a cell suspension is caused to flow into the first microchannel 18 or the second microchannel 20 and a cell layer is formed on a surface of the porous membrane 46.

### (Second Embodiment)

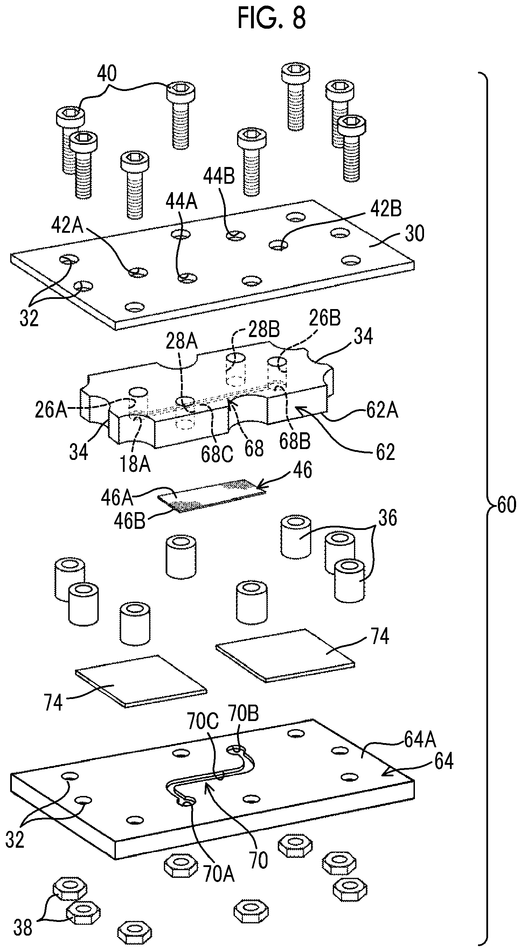

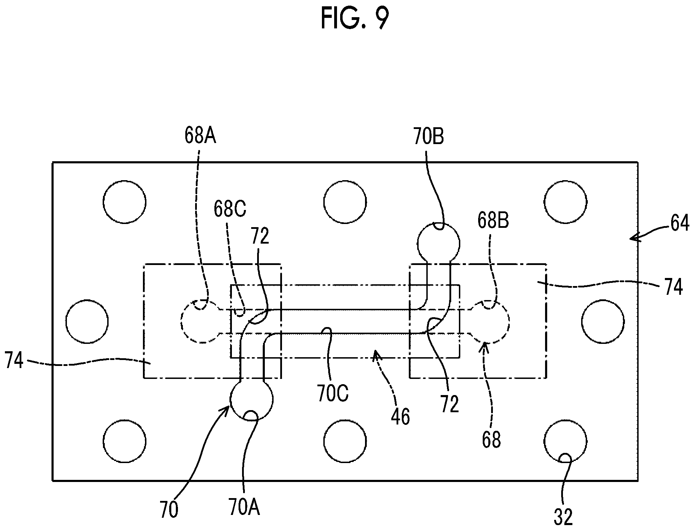

Next, a second embodiment of the present disclosure will be described with reference to Figs. 8 and 9. Note that, to facilitate the description of each configuration, the dimensions of each configuration in the drawings have been appropriately changed. Therefore, the scale of the drawings is different from that of the real scale. In addition, the same components as those in the first embodiment are given the same reference numerals and the description thereof will be omitted.

As shown in Fig. 8, as with the microchannel device 10 in the first embodiment, a microchannel device 60 in the present embodiment has a first channel member 62 in which a first microchannel 68 is formed and a second channel member 64 in which a second microchannel 70 is formed.

The first microchannel 68 and the second microchannel 70 are partitioned by the porous membrane 46 and as with the first embodiment, the first microchannel 68 and the second microchannel 70 respectively have inflow ports 68A and 70A, outflow ports 68B and 70B, and channel portions 68C and 70C through which the inflow ports 68A and 70A and the outflow ports 68B and 70B communicate with each other and that extend approximately linearly.

As shown in Fig. 9, the inflow port 68A and the outflow port 68B of the first microchannel 68 are provided at positions separated from the inflow port 70A and the outflow port 70B of the second microchannel 70 in plan view. In addition, the channel portion 68C of the first microchannel 68 is provided at a position overlapping the channel portion 70C of the second microchannel 70 in plan view.

Accordingly, step portions 72 are formed at a junction portion between the first microchannel 68 and the second microchannel 70, that is, at a position between the inflow ports 68A and 70A and the channel portions 68C and 70C and at a position between the outflow ports 68B and 70B and the channel portions 68C and 70C, respectively. Meanwhile, in the present embodiment, the width of the channel portion 68C of the first microchannel 68 is approximately the same as the width of the channel portion 70C of the second microchannel 70.

As shown in Figs. 1 to 5, a pair of reinforcing members 74 having a higher stiffness than the porous membrane 46 is disposed between the porous membrane 46 and a facing surface 64Aof the second channel member 64. For example, the pair of reinforcing members 74 is a rectangular membrane member formed of polyethylene terephthalate or the like and the thickness of the pair of reinforcing members 74 is equal to or greater than 12 µm and is smaller than the depth of each of the first microchannel 18 and the second microchannel 20.

In addition, the pair of reinforcing members 74 has a size that covers portions of the porous membrane 46 that respectively face the step portions 72 formed between the first microchannel 68 and the second microchannel 70. Furthermore, the pair of reinforcing members 74 is disposed with a gap formed therebetween and the channel portion 68C of the first microchannel 68 and the channel portion 70C of the second microchannel 70 are positioned between the pair of reinforcing members 74.

### <Action and Effect>

According to the present embodiment, the step portions 72 are formed at a junction portion between the first microchannel 68 and the second microchannel 70 and the portions of the porous membrane 46 that face the step portions 72 are reinforced by being respectively covered by the pair of reinforcing members 74.

Therefore, even in a case where a cell suspension is caused to flow into the first microchannel 68 and the liquid pressure of the cell suspension is applied to the porous membrane 46 in the case of formation of a cell layer on the upper surface 46A of the porous membrane 46, the porous membrane 46 can be restrained from being bent toward the second microchannel 70 side since the reinforcing members 74 are provided. Accordingly, it is possible to suppress formation of a gap between the step portions 72 and the porous membrane 46 and thus it is possible to restrain cells, red blood cells, or a tracer from flowing into the gap.

Furthermore, the pair of reinforcing members 74 is disposed with a gap formed therebetween and the channel portion 68C of the first microchannel 68 and the channel portion 70C of the second microchannel 70 are positioned between the pair of reinforcing members 74. Therefore, in the case of a permeability test for cells, red blood cells, or a tracer, it is possible to restrain the reinforcing members 74 from inhibiting the cells, red blood cells, or the tracer moving between the first microchannel 68 and the second microchannel 70.

### (Other Embodiments)

Although an example of the embodiments of the present disclosure has been described above, the present disclosure is not limited to the above and various modifications can be made without departing from the scope of the invention.

For example, in the first and second embodiments, the reinforcing members 54 and 74 are provided between the porous membrane 46 and the facing surfaces 14A and 64A of the second channel members 14 and 64. However, the reinforcing members 54 and 74 may be provided between the facing surfaces 12A and 62A of the first channel members 12 and 62 and the porous membrane 46.

In addition, in the first and second embodiments, the holding plate 30 is provided above the first channel members 12 and 62 and the holding plate 30 and the second channel members 14 and 64 are bonded to each other by means of the bolts 40. However, the holding plate 30 may not be provided and the first channel members 12 and 62 and the second channel members 14 and 64 may be bonded to each other through bonding, welding, adsorption (self-adsorption) or the like.

### Examples

Hereinafter, examples and comparative examples of the present disclosure will be described. Note that, the present disclosure is not to be limitedly interpreted by the following examples.

### <Manufacture of Microchannel Device>

First, as a porous membrane, a membrane member formed of polycarbonate having holes arranged in a honeycomb shape was prepared and sterilization paper was attached to opposite surfaces of the porous membrane after the porous membrane was coated with collagen. Next, the sterilization paper on a lower surface of the porous membrane was peeled off by using tweezers, the porous membrane was placed on a second channel member in which a second microchannel was formed, and the porous membrane and the second channel member were bonded to each other.

Next, the sterilization paper on an upper surface of the porous membrane was peeled off by using tweezers, a first channel member and the second channel member are positionally aligned while observing a microscope, the first channel member in which a first microchannel was formed was placed on the porous membrane, and the porous membrane and the first channel member were bonded to each other.

In addition, a holding plate was placed on the first channel member and the holding plate and the second channel member were fastened with bolts and nuts via spacers to manufacture a base microchannel device. Note that, the width and the depth of the first microchannel of the microchannel device were 200 µm and the width and depth of the second microchannel were 400 µm.

### [Example 1]

In Example 1, a microchannel device having the same configuration as in the above-described first embodiment was manufactured. Specifically, a microchannel device obtained by causing a membrane member formed of polypropylene, of which the center portion was formed with a slit, to be interposed between a porous membrane and a second channel member of a base microchannel device as a reinforcing member, was manufactured. Note that, the size of the reinforcing member was substantially the same as a facing surface (main surface) of the second channel member and the thickness of the reinforcing member was 100 µm. Regarding the stiffness of the reinforcing member, the amount of deformation measured through an evaluation method in which a steel ball as described above was used was 0.5 mm.

### [Example 2]

In Example 2, a microchannel device having the same configuration as in the above-described second embodiment was manufactured. Specifically, a microchannel device obtained by causing a pair of membrane members formed of polyethylene terephthalate to be interposed between a porous membrane and a second channel member of a base microchannel device as a reinforcing member, was manufactured. Note that, the size of each reinforcing member was 3 mm square, and the thickness of each reinforcing member was 12 µm. Regarding the stiffness of the reinforcing member, the amount of deformation measured through an evaluation method in which a steel ball as described above was used was 1 mm. In addition, the reinforcing members were disposed at a junction portion between a first microchannel and a second microchannel.

### [Comparative Example 1]

In Comparative Example 1, no reinforcing member was disposed and a base microchannel device was used as it was.

### [Comparative Example 2]

In Comparative Example 2, a microchannel device obtained by causing a pair of membrane members formed of polyethylene terephthalate to be interposed between a porous membrane and a second channel member of a base microchannel device as a reinforcing member, was manufactured. Note that, the size of each reinforcing member was 3 mm square, and the thickness of each reinforcing member was 2 µm. In addition, the reinforcing members were disposed at a junction portion between a first microchannel and a second microchannel.

### <Observation of Cells>

Cells were seeded on the porous membranes of the microchannel devices in Example 1, Example 2, Comparative Example 1, and Comparative Example 2 and the cells were observed. Specifically, a suspension of bone marrow-derived mesenchymal stem cells (Lonza) was adjusted at a concentration of 3 × 10⁶ cells/ml and 200 µL of the suspension was injected into the second microchannels on a lower side. Next, the microchannel devices were inverted and were left for 3 hours at 37°C in a CO² incubator, a medium was caused to flow at a rate of 0.7 µL per minute, and propagation was performed overnight.

Next, a suspension of iPS cell-derived vascular endothelial cells (iCell EC manufactured by CDI) stained with CellTracker Orange (Thermo Fisher Scientific) was adjusted at a concentration of 1 × 10⁶ cells/ml and 200 µL of the suspension was injected into the first microchannels on an upper side.

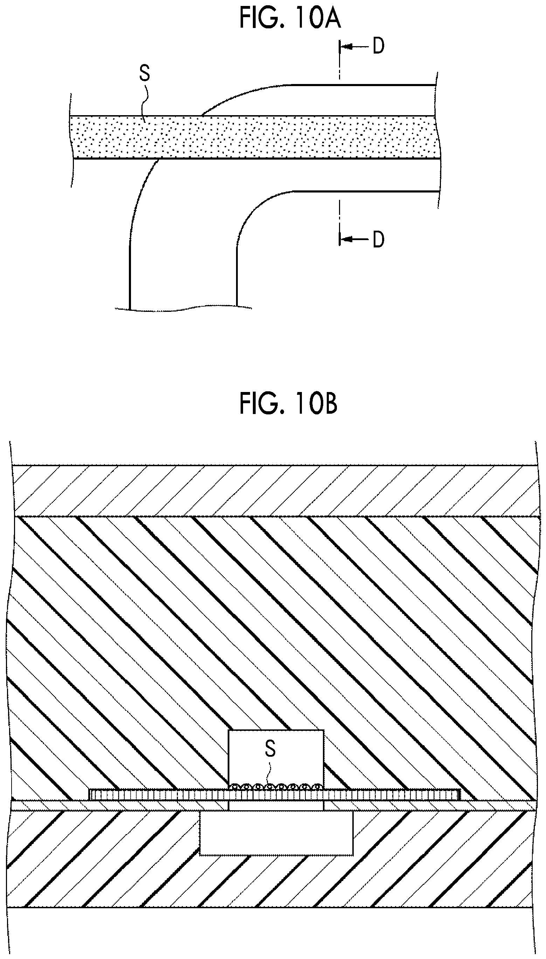

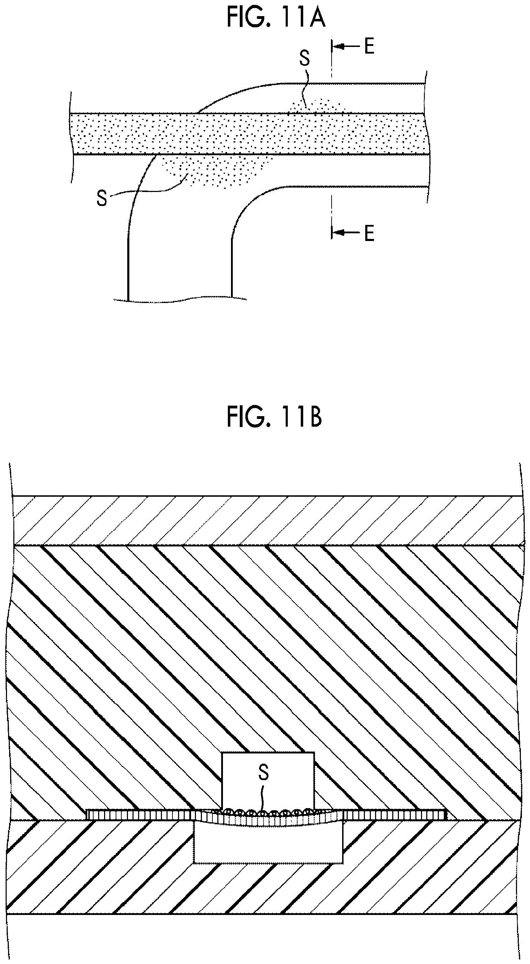

Thereafter, in the microchannel devices in Example 1, Example 2, Comparative Example 1, and Comparative Example 2, the distribution of the iPS cell-derived vascular endothelial cells injected into each of the first microchannels was observed using a fluorescence microscope. The result of observation of Example 1 is shown in Figs. 10A and 10B, and the result of observation of Comparative Example 1 is shown in Figs. 11A and 11B. Note that, in Figs. 10A, 10B, 11A, and 11B, only the iPS cell-derived vascular endothelial cells are shown.

In the case of Example 1, as shown in Figs. 10A and 10B, it was observed that iPS cell-derived vascular endothelial cells S remained in the first microchannel. On the other hand, in the case of Comparative Example 1, as shown in Figs. 11A and 11B, it was observed that a portion of the iPS cell-derived vascular endothelial cells S was positioned in the second microchannel and thus the iPS cell-derived vascular endothelial cells S seemed to have leaked into the second microchannel.

Note that, although not shown, the results of observation of Example 2 and Comparative Example 2 were similar to those of Example 1 and Comparative Example 1. Specifically, the iPS cell-derived vascular endothelial cells S remained in the first microchannel in the case of Example 2 and in the case of Comparative Example 2, it was observed that a portion of the iPS cell-derived vascular endothelial cells S was positioned in the second microchannel and thus the iPS cell-derived vascular endothelial cells S seemed to have leaked into the second microchannel.

The entire disclosure of Japanese Patent Application No. 2018-037511 filed on Mar. 2, 2018, is incorporated herein by reference.

All publications, patent applications, and technical standards described in the present specification are incorporated herein by reference to the same extent as if each publication, patent application, or technical standard was specifically and individually indicated to be incorporated by reference.

### Explanation of References

- 10, 60:: microchannel device
- 12, 62:: first channel member
- 12A, 62A:: facing surface
- 14, 64:: second channel member
- 14A, 64A:: facing surface
- 16:: channel unit
- 18, 68:: first microchannel
- 18A, 68A:: inflow port
- 18B, 68B:: outflow port
- 18C, 68C:: channel portion
- 20, 70:: second microchannel
- 20A, 70A:: inflow port
- 20B, 70B:: outflow port
- 20C, 70C:: channel portion
- 22, 24, 72:: step portion
- 26A, 26B, 28A, 28B:: through-hole
- 30:: holding plate
- 32, 56:: bolt hole
- 34:: recess portion
- 36:: spacer
- 38:: nut
- 40:: bolt
- 42A, 42B, 44A, 44B:: through-hole
- 46:: porous membrane
- 46A:: upper surface
- 46B:: lower surface
- 48:: hole
- 48A:: opening
- 50:: flat portion
- 52:: communication hole
- 54, 74:: reinforcing member
- 57:: through-hole
- 58:: slit

## Claims

1. A microchannel device comprising:
a first microchannel that is formed in a first channel member;
a second microchannel that is formed in a second channel member and at least a portion of which overlaps the first microchannel in plan view, the second microchannel having a step portion formed between the first microchannel and the second microchannel;
a porous membrane that has a plurality of holes penetrating the porous membrane in a thickness direction and is disposed between the first channel member and the second channel member to partition the first microchannel and the second microchannel; and
a reinforcing member that is provided between the first channel member or the second channel member and the porous membrane, is higher in stiffness than the porous membrane, and reinforces at least a portion of the porous membrane that faces the step portion.

2. The microchannel device according to claim 1,
wherein the first microchannel and the second microchannel are partially separated from each other in plan view, and
wherein the step portion is formed at a junction portion at which the first microchannel and the second microchannel join each other in plan view.

3. The microchannel device according to claim 1,
wherein a width of the first microchannel is smaller than a width of the second microchannel, and
wherein the step portion is formed by a difference between the width of the first microchannel and the width of the second microchannel.

4. The microchannel device according to any one of claims 1 to 3,
wherein the reinforcing member has a size that covers the entire porous membrane, and
wherein a slit is formed in the reinforcing member at a portion where the porous membrane faces the first microchannel or the second microchannel.

5. The microchannel device according to claim 4,
wherein a width of the slit of the reinforcing member is equal to or smaller than a width of each of the first microchannel and the second microchannel.

6. The microchannel device according to any one of claims 1 to 5,
wherein the reinforcing member is a membrane member formed of polyethylene terephthalate.

7. The microchannel device according to any one of claims 1 to 5,
wherein the reinforcing member is a membrane member formed of polypropylene.

8. The microchannel device according to any one of claims 1 to 7,
wherein a thickness of the reinforcing member is equal to or greater than 12 µm.

9. The microchannel device according to any one of claims 1 to 8,
wherein a thickness of the reinforcing member is smaller than a depth of each of the first microchannel and the second microchannel.
